# EUROPEAN PATENT APPLICATION

(11) **EP 1 707 157 A1**
(43) Date of publication of application: **04.10.2006**
(21) Application number: 06251705.7
(22) Date of filing: 29.03.2006
(51) Int. Cl.: A61F 2/36

(54) **Femoral prosthetic component**

(30) Priority: 30.03.2005 GB 0506398
(71) Applicant: BENOIST GIRARD SAS, 14201 Hérouville-Saint-Clair Cédex (FR)
(72) Inventor: Bachelier, Jean-Claude, 14112 Biéville Beuville (FR)
(74) Representative: Bridge-Butler, Alan James

(57) **Abstract**

A femoral prosthetic component of a replacement hip joint comprising a stem (1) for fixing in a medullary cavity, a separate proximal element (10) provided with a neck (11) to receive a ball head or having a ball head and means for securing the proximal component to the stem which includes a sliding tongue and groove joint, the tongue (4) being provided on the stem by an undercut rail which extends in a medial-lateral direction the groove (14) being provided in the separate proximal element, and the tongue and groove being tapered along their lengths.

## Description

This invention relates to a femoral prosthetic component for a replacement hip joint which comprises a stem for fixing in a medullary canal, separate proximal element provided with a neck to receive a ball head or having a ball head and means for securing the proximal component to the stem which will enable a modular construction so that stems and heads of different sizes and/or shapes can be mated together.

French Patents Nos. 2 626 168 and 2 721 200 both show femoral prosthetic components which have separate stems and proximal elements which can be fastened together but both constructions show the use of a tapered plug provided on the separate proximal element and which is located in a socket in the stem. Such constructions are expensive to produce and it can be difficult to remove the plug from the stem once it has been placed in position.

US 5,336,268 shows an adjustable hip joint endoprosthesis which has an adjustable prosthesis head which includes a link element which can slide in an undercut groove on the stem. The position of the link element can be adjusted in a medial/lateral direction and is locked in position by a threaded tension pin.

US 5,800,560 also shows an adjustable hip joint prosthesis which is multi-dimensionally adjusted on the neck portion and which includes a dovetail shaped groove in a plate which can be secured to the stem and on which is a second plate which is movable in relation to the first and which also carries a second dovetail shaped groove which is normal to the direction of the first and which carries a tapered cone to receive a ball head. Thus the construction allows adjustment in two planes and can be firmly fastened in any position by means, for example, of screws.

Neither of the above US Patent specifications shows a construction in which the undercut grooves are tapered so that the stem and separate proximal element are accurately located in a set position by the use of tapers.

The present invention is intended to provide an improved construction which is easier for the surgeon to operate.

According to the present invention a femoral prosthetic component of a replacement hip joint comprises a stem for fixing in a medullary cavity, a separate proximal element provided with a neck to receive a ball head or having a ball head and means for securing the proximal component to the stem which include a sliding tongue and groove joint, the tongue being provided on the stem by an undercut rail which extends in a medial-lateral direction the groove being provided in the separate proximal element, and the tongue and groove being tapered along their lengths.

Thus, with this construction it is easy for the surgeon to slide the proximal element into place, and their tapered lengths allow the parts to wedge together, and it is also relatively easy to remove the proximal element if an alternative element is required by releasing the wedging action of the tapers.

Preferably the widths and/or depths of the tongue and groove are tapered along their lengths.

In one preferred construction the opposed side walls of the tongue can be inclined to each other provide a dovetail shape.

In another convenient construction the tongue and groove have co-operating cross-sections which are substantially T-shaped.

The cross arms of the T-shapes can have downwardly projecting extensions to engage co-operating troughs in the groove.

Means for releasably locking the stem and proximal element together be provided, for example, by means of a set screw.

The tongue can be provided as first and second lengths with a gap between them and the groove can also have first and second lengths with a gap between them to allow the grooves to be located on the tongue at an intermediate position in the length thereof and then moved lengthwise to the final located position.

The stem and/or proximal element can be made from metal or a composite synthetic material.

If a separate ball head is provided this can be made of metal, a synthetic material or a ceramic material.

The invention also includes a kit of parts to provide a modular construction of the femoral prosthetic component as set forth above and which includes two or more alternative stems and/or two or more alternative proximal elements which are of different sizes and/shapes and which have appropriate sliding tongue and groove joints which can be assembled together to form said prosthetic component.

The invention can be performed in various ways and some embodiments will now be described by way of example and with reference to the accompanying drawings in which :
Figure 1 is a side elevation of a femoral prosthetic component according to the present invention;
Figure 2 is an isometric view of the separate proximal element and part of the stem of the kind shown in Figure 1 and ready for assembly together;
Figure 3 is a plan view of the upper end of the stem shown in Figures 1 and 2;
Figure 4 is an isometric view showing the use of means for locking the parts together;
Figure 5 is a part cross-sectional elevation on lines V-V on Figure 4;
Figure 6 is an exploded view showing an alternative construction;
Figure 7 is an isometric view of a stem utilising a T-shaped tongue rail; and,
Figure 8 is a part cross-sectional elevation of the stem as shown in in Figure 6 secured to a separate proximal element.

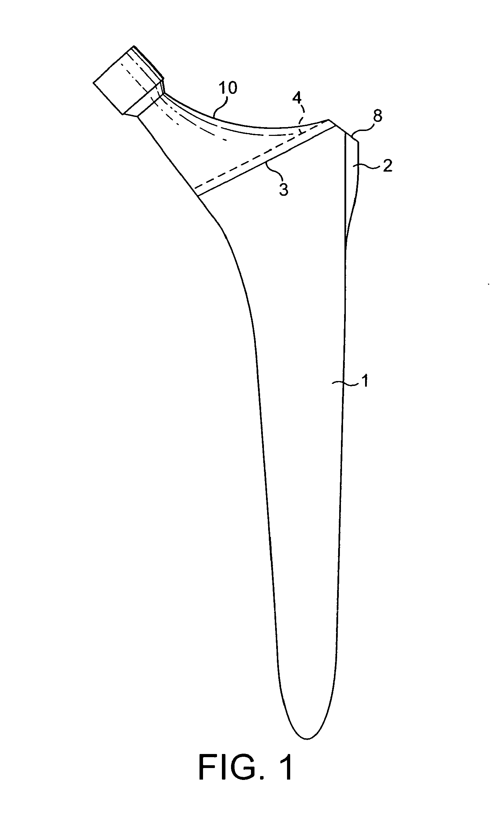

As shown in Figures 1 to 3 of the drawings a femoral prosthetic component of a replacement hip joint according to the present invention comprises a stem 1 for fixing in a medullary cavity. The stem can be of any suitable size or shape and can be made from metal, for example a stainless steel or a composite synthetic material. The proximal end of the stem has a fin 2 on its lateral side to assist location.

The proximal end of the stem is shaped to provide a flat platform 3 on which is located a wedge-shaped undercut rail 4. The rail 4 tapers in width and depth from its medial to its lateral end and is undercut so that the cross-sectional width of its upper end 5 is greater than the cross-sectional width of its base 6. The opposed side walls 7 of the rail are inclined to each other to provide a dovetail shape and rail 4 provides a tongue for a tongue and groove joint.

The upper lateral corner of the stem 1 is chamfered as indicated by reference numeral 8.

The separate proximal element 10 for use with the stem 1 comprises a neck portion 11 and a tapered spigot 12 to receive a ball head (not shown) in known manner. Ball heads of this type are provided with a tapered socket to mate with the spigot 12.

In an alternative construction (not shown) the ball head could be integral with the proximal element 10. If a separate ball head is provided it could be made of metal, a synthetic material or ceramic material.

The proximal element 10 has a flat planar base 13 in which is provided a tapered groove 14. The walls of the groove and the dimensions of the taper are arranged so that the groove is a sliding fit over the tongue provided by the rail 4 and the tapering sides of the walls 15 of the groove mate with the side walls 7 of the rail to provide a dove tail tongue and groove joint. It will be seen that the lengthwise taper of the tongue is in a medial-lateral direction and the taper can be a Morse taper so that when the two parts are pushed into position they tend to wedge together, the inclined opposed side walls of the joint preventing any inclination to come part and again providing a taper locking connection.

If it is desired to take the parts apart it is merely necessary to apply a sharp blow to one of the parts in the appropriate direction to release them from the tapers.

In the construction described above the rail is tapered in both width and depth but, alternatively, it could only be tapered in one or the other.

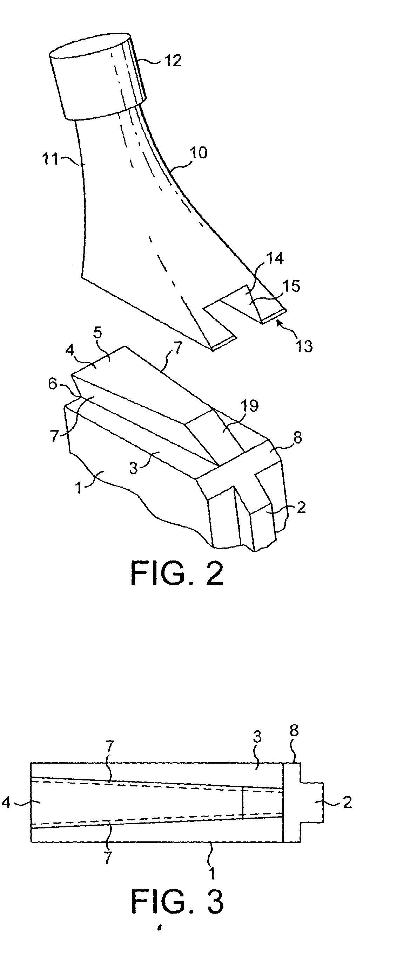

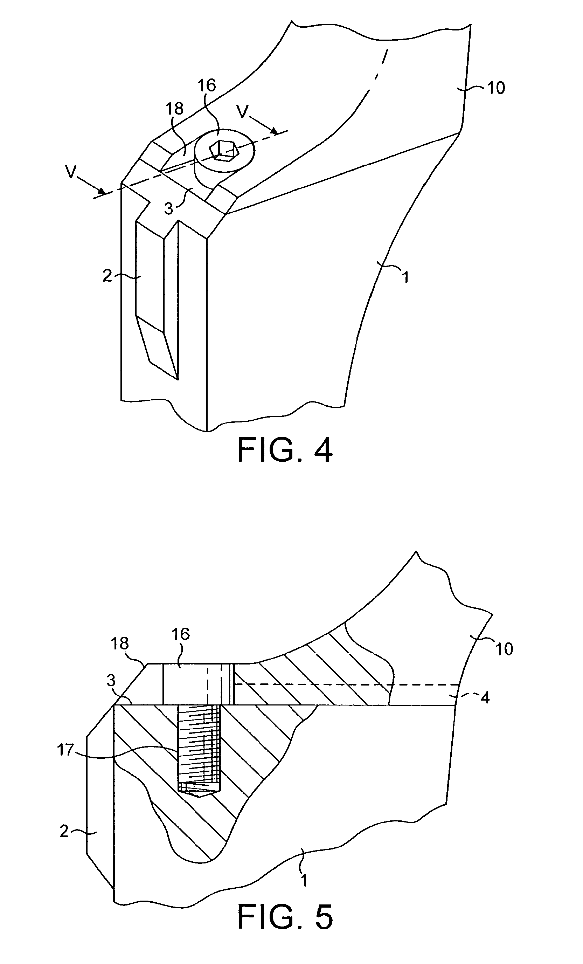

Figures 4 and 5 show a construction in which the same reference numerals are used to indicate similar parts to those shown in Figures 1 to 3. In this arrangement means are included for releasably locking the separate proximal element together and to enhance the locking effect of the tapers on the tongue and groove joint. This is achieved by use of a set screw 16 which is screwed into a threaded bore 17 in the stem. The lower part of the separate proximal element 10 is cut away to provide a recess 18 in which the head of the set screw is located. The set screw 16 therefore acts as a stop to prevent the proximal element 10 sliding backwards and detaching from the stem 1. As will be seen from Figure 2 the medial end of the undercut rail 4 is provided with a chamfer 19. This chamfer is omitted in the construction shown in Figures 4 and 5 and is replaced by the threaded bore 17.

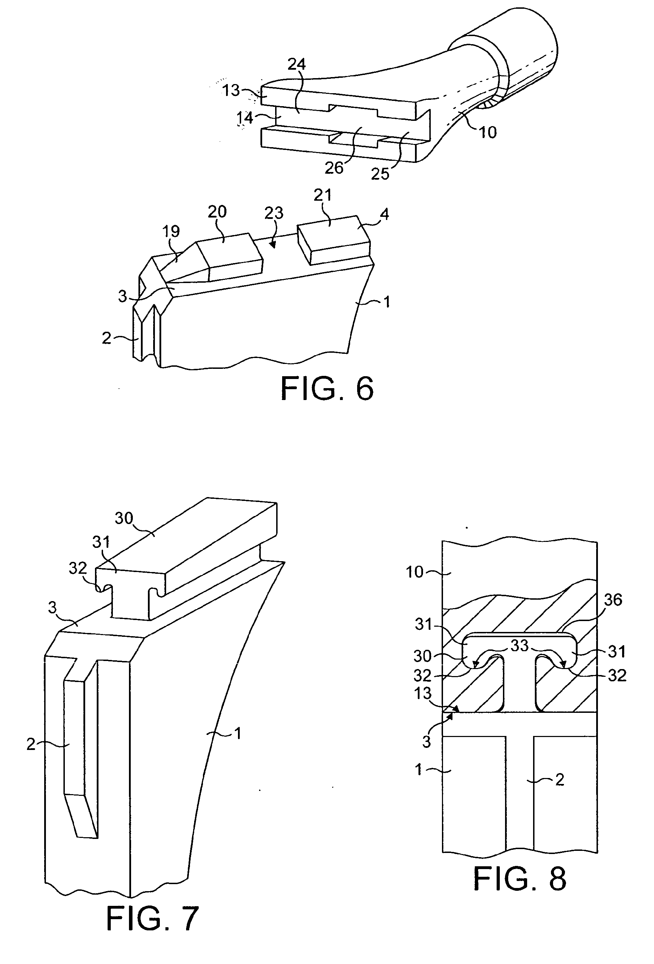

Figure 6 shows the stem 1 and separate proximal element 10 and the same reference numerals are used to indicate similar parts as in Figures 1 to 3. In this arrangement however the undercut rail 4 which acts as the tongue is provided as a first medial-lateral length 20 and a second medial-lateral length 21 with a gap 23 between them. Similarly the tapered groove 14 in the separate proximal element 10 is also formed with a first medial-lateral length 24 and a second medial-lateral length 25 with a gap 26 between them. The dimensions of the gap 26 are arranged to be slightly larger than the length of the first length 20 of the tongue and gap 23 in the tongue is arranged to be slightly larger than the walls of the second length 25 of the groove so that the proximal element 10 can be placed downwardly over the stem 1 until flat planar base 13 of the proximal element engages the flat platform 3 of the stem with the proximal element located approximately halfway along the rail 4. The proximal element can now be moved in a medial-lateral direction to fully engage the rail in the groove and provide the tongue and groove connection.

This construction enables the parts to be put together after the stem has been inserted in the bone and requires a short medial-lateral distance for engagement. Thus it will be appreciated that the medial-lateral dimension required is only half that of the construction shown in Figures 1, 2 and 3.

Figures 7 and 8 show another alternative construction and in which the same reference numerals are used to indicate similar parts to those shown in Figures 1, 2 and 3. In this construction however the tapered dovetail shaped rail 4 of the construction shown in Figures 1, 2 and 3 is replaced by a rail 30 which is of T-shaped cross-section. The cross arms 31 of the T have downwardly projecting extensions 32 with rounded extremities. The T-shaped rail is again tapered in depth and width along its medial-lateral length.

The proximal element 10 is provided with a T-shaped co-operating groove 35 which is shaped and dimensioned to engage the tapered T-shaped rail 30 and lock in position in a similar manner to that described with regard to the dovetail shaped rail 4 in the other constructions. The groove 35 is relieved at 36 to provide a small space between the top of the T of the rail and the bottom of the groove 35 and the lower corners are chamfered as will be seen from Figure 8. With this arrangement the flat planar base 13 of the proximal element 10 is arranged to engage the flat platform 3 on the stem 1. The rounded extremities of the downwardly projecting extensions 32 of the cross arms 31 engage suitably shaped rounded troughs 33 in the groove 35 so that the wedging effect acting on the proximal element 10 is between the troughs 33 and the flat planar base 13.

As mentioned above the groove 35 and rail 30 are tapered in a similar manner to the construction shown in the other Figures and the taper can again be a Morse taper so that when the two parts are pushed into position they tend to wedge together.

If required the positive stop provided by the set screw 16 can also be provided in the construction shown in Figures 7 and 8 and similarly the T-shaped rail 31 and groove 35 can be provided with gaps in a similar manner to the construction shown in Figure 6 to reduce the dimensions required to achieve engagement.

The invention provides a kit of parts which can include a number of stem elements and a number of proximal elements which could have different shapes and sizes and all of which have the sliding tongue and groove joint so that any stem can be connected to any proximal element to provide the shape required by the surgeon.

The separate proximal element can be made from any suitable material, for example metal or a synthetic plastics material.

Due to the modular arrangement proximal elements can be used which can be angled or shaped to fit the requirements of the patient and can be fitted to a suitable length or thickness of stem. This provides a very large variety of shapes and sizes.

The made up component can be used for revision surgery or the initial fitting of a prosthesis.

## Claims

1. A femoral prosthetic component of a replacement hip joint comprising a stem for fixing in a medullary cavity, a separate proximal element provided with a neck to receive a ball head or having a ball head and means for securing the proximal component to the stem which includes a sliding tongue and groove joint, the tongue being provided on the stem by an undercut rail which extends in a medial-lateral direction the groove being provided in the separate proximal element, and the tongue and groove being tapered along their lengths.

2. A femoral prosthetic component as claimed in claim 1 in which the widths and/or depths of the tongue and groove are tapered along their lengths

3. A femoral prosthetic component as claimed in claim 1 or claim 2 in which the opposed side walls of the tongue are inclined to each other to provide a dove tail shape.

4. A femoral prosthetic component as claimed in claim 1 or claim 2 in which the tongue and groove have co-operating cross-sections which are substantially T-shaped.

5. A femoral prosthetic component as claimed in claim 2 in which the cross-section of the T-shapes have downwardly projecting extensions to engage co-operating troughs in the groove.

6. A femoral prosthetic component as claimed in claims 1 to 5 including means for releasably locking the stem and proximal element together.

7. A femoral prosthetic component as claimed in claim 6 in which the locking means include a set screw.

8. A femoral prosthetic component as claimed in claims 1 to 7 in which the tongue is provided as first and second lengths with a gap between them and the groove also has first and second lengths with a gap between them to allow the grooves to be located on the tongue at an intermediate position in the length thereof and then moved lengthwise to the final located position.

9. A femoral prosthetic component as claimed in any one of the preceding claims in which the stem and/or proximal element are made from metal or a composite synthetic material.

10. A femoral prosthetic component as claimed in any one of the preceding claims in which a separate ball head is provided which is made of metal, a synthetic material or ceramic material.

11. A femoral prosthetic component as set forth in any one of the preceding claims which provides a modular construction and which is in the form of a kit of parts which includes two or more alternative stems and/or two or more alternative proximal elements which are of different sizes and/or shapes and which have appropriate sliding tongue and groove joints which can be assembled together to form said prosthetic component.
